# EUROPÄISCHE PATENTSCHRIFT

(11) **EP 0 811 610 B1**
(45) Veröffentlichungstag und Bekanntmachung des Hinweises auf die Patenterteilung: **28.11.2001**
(21) Anmeldenummer: 97108258.1
(22) Anmeldetag: 22.05.1997
(51) Int. Cl.: C07C 279/22, A61K 31/155

(54) **Ortho-substituierte Benzoylguanidine, Verfahren zu ihrer Herstellung, ihre Verwendung als Medikament oder Diagnostikum sowie sie enthaltendes Medikament**
Ortho-substituted benzoylguanidines process for their preparation, their use as medicament or diagnostic agent and medicaments containing them
Benzoylguanidines ortho-substituées, procédé pour leur préparation, leur utilisation comme médicament ou agent de diagnostique et médicaments les contenant

(30) Priorität: 04.06.1996 DE 19622370
(43) Veröffentlichungstag der Anmeldung: 10.12.1997
(73) Patentinhaber: HOECHST AKTIENGESELLSCHAFT, 65929 Frankfurt am Main (DE)
(72) Erfinder: Weichert, Andreas, Dr., 63329 Egelsbach (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Schwark, Jan-Robert, Dr., 65779 Kelkheim (DE); Albus, Udo, Dr., 61197 Florstadt (DE); Scholz, Wolfgang, Dr., 65760 Eschborn (DE)

(56) Entgegenhaltungen:
- EP-A- 0 612 723
- EP-A- 0 628 543
- EP-A- 0 690 048

## Beschreibung

Die Erfindung betrifft Benzoylguanidine der Formel I 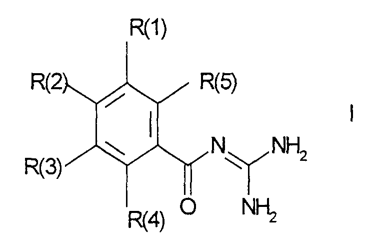 worin bedeuten:
- R(1): H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CF₂)_{c}-CF₃;
X Sauerstoff oder Schwefel;
a Null oder 1;
c Null, 1, 2 oder 3;
oder
- R(1): -SR(10) oder -OR(10);
R(10) -C_{f}H_{2f}-Cycloalkyl mit 3, 4, 5, 6, 7 oder 8 C-Atomen im Cycloalkylring, Heteroaryl mit 1, 2, 3, 4, 5, 6, 7, 8 oder 9 C-Atomen oder Phenyl,
wobei Heteroaryl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
f Null oder 1;
oder
- R(1): Phenyl, Naphthyl, Biphenylyl oder Heteroaryl mit 1, 2, 3, 4, 5, 6, 7, 8 oder 9 C-Atomen, letzteres über ein C- oder N-Atom des Rings verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
- R(1): -SR(13), -OR(13), -NHR(13), -NR(13)R(14), -C≡CR(18) oder -C[R(19)]=CHR(18);
R(13) und R(14) gleich oder verschieden -(CH₂)_{g}-(CHOH)ₕ-(CH₂)ᵢ-(CHOH)ₖₖ-R(17) oder -(CH₂)_{g}-O-(CH₂-CH₂O)ₕ-R(24);
R(17) Wasserstoff oder Methyl,
g, h und i
gleich oder verschieden Null, 1 oder 2;
kk 1 oder 2;
R(24) H, Alkyl mit 1, 2, 3, 4, 5 oder 6 C-Atomen, Cycloalkyl mit 3, 4, 5, 6, 7 oder 8 C-Atomen oder -CₘH₂ₘ-R(18);
m 1, 2, 3 oder 4;
R(18) Phenyl,
das unsubstituiert oder substituiert ist mit 1 - 3 Substituenten ausgewählt aus der Gruppe bestehend aus F, Cl, CF₃, Methyl, Methoxy und NR(25)R(26); R(25) und R(26)
H oder Alkyl mit 1, 2, 3 oder 4 C-Atomen; oder
R(18) Heteroaryl mit 1, 2, 3, 4, 5, 6, 7, 8 oder 9 Kohlenstoffatomen, das unsubstituiert oder wie Phenyl substituiert ist; oder
R (18) Cycloalkyl mit 3, 4, 5, 6, 7 oder 8 C-Atomen;
R(19) Wasserstoff oder Methyl; einer der Substituenten R(2) und R(3)
-O-CO-R(27);
R(27) Alkyl mit 1, 2, 3 oder 4 C-Atomen, Cycloalkyl mit 3, 4, 5 oder 6 C-Atomen, Phenyl, Biphenylyl, Naphthyl, Pyridyl oder Chinolinyl,
wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, CF₃, Methyl und Methoxy;
und
der jeweils andere der Substituenten R(2) und R(3)
definiert ist wie R(1);
- R(4) und R(5): Wasserstoff, Alkyl mit 1 oder 2 C-Atomen, Alkoxy mit 1 oder 2 C-Atomen, F, Cl, CN oder -(CF₂)ₒ-CF₃,
O Null oder 1
wobei R(4) und R(5) nicht gleichzeitig Wasserstoff sind;
sowie deren pharmazeutisch verträgliche Salze.

Bevorzugt sind Verbindungen der Formel I, in denen bedeuten:
- R(1): H, F, Cl, Alkyl mit 1, 2, 3 oder 4 C-Atomen, Cycloalkyl mit 5 oder 6 C-Atomen oder -Xₐ-CF₃;
X Sauerstoff;
a Null oder 1;
oder
- R(1): -SR(10) oder -OR(10);
R(10) Cycloalkyl mit 4, 5 oder 6 C-Atomen, Chinolyl, Isochinolyl, Pyridyl oder Phenyl,
wobei Chinolyl, Isochinolyl und Pyridyl über ein C- oder ein N-Atom ihres Rings gebunden sind,
und wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
- R(1): Chinolyl, Isochinolyl oder Pyridyl,
die über ein C- oder N-Atom ihres Rings verknüpft sind,
oder
- R(1): -C≡CR(18);
R(18) Phenyl oder Cycloalkyl mit fünf oder 6 C-Atomen;
- R(2) und R(3): -O-CO-R(27);
R(27) Alkyl mit 1, 2, 3 oder 4 C-Atomen, Cycloalkyl mit 3, 4, 5 oder 6 C-Atomen, Phenyl, Biphenylyl, Naphthyl, Pyridyl oder Chinolinyl,
wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, CF₃, Methyl und Methoxy; wobei stets einer der Substituenten R(2) und R(3)
definiert ist wie R(1);
R(4) und R(5) unabhängig voneinander Methyl, Methoxy, F, Cl, CN oder CF₃.

Besonders bevorzugt sind Verbindungen der Formel I, in denen bedeuten:
- R(1): H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder CF₃;
einer der Substituenten R(2) und R(3)
-O-CO-CH₃;
und
der jeweils andere der Substituenten R(2) und R(3)
definiert ist wie R(1);
- R(4)und R(5): Methyl, Methoxy, F, Cl, CN oder CF₃,
sowie deren pharmazeutisch verträgliche Salze.

Unter Heteroaryl mit 1, 2, 3, 4, 5, 6, 7, 8 oder 9 C-Atomen werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.
Als Heteroaryl mit 1, 2, 3, 4, 5, 6, 7, 8 oder 9 C-Atomen gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl; besonders Furanyl, Thienyl, Pyrrolyl, Imidazolyl, Pyrazolyl, Triazolyl, Thiazolyl, Pyridyl, Indolyl, Chinolyl und Isochinolyl.

Enthält einer der Substituenten R(1) bis R(5) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können geradkettig oder verzweigt sein.

Die Erfindung betrifft weiterhin ein Verfahren zur Herstellung der Verbindung I, dadurch gekennzeichnet, daß man eine Verbindung der Formel II 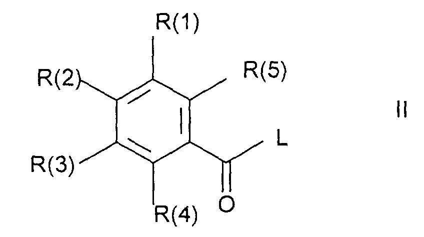 worin R(1) bis R(5) die angegebene Bedeutung besitzen und L für eine leicht nucleophil substituierbare Fluchtgruppe steht,
mit Guanidin umsetzt.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäure-derivaten (Formel II, L = OH) herstellen, wie die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂ H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafl uorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20□ C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 2-, 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Den Verbindungen I ähnliche Verbindungen sind bekannt aus der Europäischen Offenlegungsschrift 612 723 A 1 (HOE 93/F 054). Diese enthalten bereits Hydroxylgruppen als Substituenten im Phenylkern, jedoch keine Substituenten in ortho-Stellung. Ähnliche Verbindungen sind auch aus EP 628 543 und EP 690 048 bekannt, jedoch findet sich darin nirgendwo die Gruppe -O-CO-R(27) in Positionen von R(2) oder R(3).

Gegenüber den bekannten Verbindungen zeichnen sich die erfindungsgemäßen Verbindungen durch eine außerordentlich hohe Wirksamkeit in der Inhibition des Na⁺/H⁺-Austauschs aus, sowie durch eine verbesserte Wasserlöslichkeit aus

Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheiten wichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Es wurde außerdem gefunden, daß Verbindungen der Formel I eine günstige Beeinflussung der Serumlipoproteine zeigen. Es ist allgemein anerkannt, daß für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter Serum-Lipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken.. Es wurde nun gefunden, daß Verbindungen der Formel I bezüglich der Beeinflussung der Serumlipidspiegel wertvolle therapeutisch verwertbare Eigenschaften zeigen. So erniedrigen sie signifikant die erhöhten Serum Konzentration von LDL und VLDL, wie sie beispielweise durch erhöhte dietätische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen die Verbindungen der Formel I zu einer deutlichen Reduktion der durch Stoffwechselanomalien induzierten Infarkte und insbesondere zu einer signifikanten Verminderung der induzierten Infarktgröße und dessen Schweregrades. Weiterhin führen Verbindungen der Formel I zu einen wirksamen Schutz gegen durch Stoffwechselanomalien induzierte Endothelschädigungen. Mit diesem Schutz der Gefäße gegen das Syndrom der endothelialen Dysfunktion sind Verbindungen der Formel I wertvolle Arzneimittel zur Prävention und zur Behandlung koronarer Gefäßsspasmen, der Atherogenese und der Atherosklerose, der linksventrikulären Hypertrophie und der dilatierten Kardiomyopathie, und thrombotischer Erkrankungen.

Die genannten Verbindungen finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten; die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter cardialer Hypertrophien und Cardiomyopathien, zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten, eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, erweist sich als günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:
- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq.: Äquivalent

### Experimenteller Teil

### Allgemeine Vorschrift zur Herstellung von Benzoyl-guanidinen (I) Variante A: aus Benzoesäuren (II, L=OH)

1.0 eq. des Benzoesäurederivates der Formel II löst bzw. suspendiert man in wasserfreiem THF ( 5 ml/mmol ) und versetzt sodann mit 1.1 eq.

Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCI auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I) Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Sieden erhitzt (typische Reaktionszeit 2 bis 5 h). Das Lösungsmittel wird unter vermindertem Druck (Rotationsverdampfer) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: 4-Acetyloxy-2-chloro-5-trifluormethyl-benzoylguanidin-hydrochlorid:

Farblose Kristalle M⁺+H= 324
aus 2-Chloro-4-hydroxy-5-trifluormethyl-benzoylguanidin durch Acetylierung mittels Acetylchlorid in Gegenwart von Cäsiumcarbonat in NMP und anschließender Hydrochloridbildung nach Variante A.

### Beispiel 2: 4-Acetyloxy-2-methoxy-5-trifluormethyl-benzoylguanidinhydrochlorid:

Farblose Kristalle M⁺+H= 320
aus 4-Hydroxy-2-methoxy-5-trifluormethyl-benzoylguanidin durch Acetylierung mittels Acetylchlorid in Gegenwart von Cäsiumcarbonat in NMP und anschließender Hydrochloridbildung nach Variante A.

### Beispiel 3: 3-Acetyloxy-2-methoxy-5-tert.butyl-benzoylguanidin-hydrochlorid:

Farblose Kristalle M⁺+H= 308
aus 3-Hydroxy-2-methoxy-5-tert.butyl-benzoylguanidin durch Acetylierung mittels Acetylchlorid in Gegenwart von Cäsiumcarbonat in NMP und anschließender Hydrochloridbildung nach Variante A.

## Patentansprüche

1. Ortho-substituierte Benzoylguanidine der Formel I 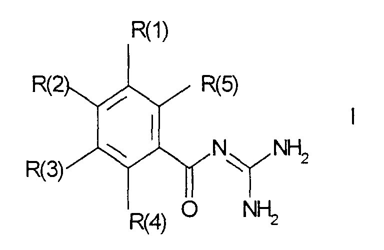 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CF₂)_{c}-CF₃;
X Sauerstoff oder Schwefel;
a Null oder 1;
c Null, 1, 2 oder 3;
oder
R(1) -SR(10) oder -OR(10);
R(10) -C_{f}H_{2f}-Cycloalkyl mit 3, 4, 5, 6, 7 oder 8 C-Atomen im Cycloalkylring, Heteroaryl mit 1, 2, 3, 4, 5, 6, 7, 8 oder 9 C-Atomen oder Phenyl,
wobei Heteroaryl und Phenyl unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino;
f Null oder 1;
oder
R(1) Phenyl, Naphthyl, Biphenylyl oder Heteroaryl mit 1, 2, 3, 4, 5, 6, 7, 8 oder 9 C-Atomen, letzteres über ein C- oder N-Atom des Rings verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14), -C≡CR(18) oder -C[R(19)]=CHR(18);
R(13) und R(14) gleich oder verschieden -(CH₂)_{g}-(CHOH)ₕ-(CH₂)ⱼ-(CHOH)ₖₖ₋ R(17) oder -(CH₂)_{g}-O-(CH₂-CH₂O)ₕ-R(24);
R(17) Wasserstoff oder Methyl,
g, h und i
gleich oder verschieden Null, 1 oder 2;
kk 1 oder 2;
R(24) H, Alkyl mit 1, 2, 3, 4, 5 oder 6 C-Atomen, Cycloalkyl mit 3, 4, 5, 6, 7 oder 8 C-Atomen oder-CₘH₂ₘ-R(18);
m 1, 2, 3 oder 4;
R(18) Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen, das unsubstituiert oder wie Phenyl substituiert ist;
oder
R (18) Cycloalkyl mit 3, 4, 5, 6, 7 oder 8 C-Atomen;
R(19) Wasserstoff oder Methyl;
einer der Substituenten R(2) und R(3)
-O-CO-R(27);
R(27) Alkyl mit 1, 2, 3 oder 4 C-Atomen, Cycloalkyl mit 3, 4, 5 oder 6 C-Atomen, Phenyl, Biphenylyl, Naphthyl, Pyridyl oder Chinolinyl,
wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, CF₃, Methyl und Methoxy;
und
der jeweils andere der Substituenten R(2) und R(3)
definiert ist wie R(1);
R(4) und R(5) Wasserstoff, Alkyl mit 1 oder 2 C-Atomen, Alkoxy mit 1 oder 2 C-Atomen, F, Cl, CN oder -(CF₂)ₒ-CF₃,
O Null oder 1
wobei R(4) und R(5) nicht gleichzeitig Wasserstoff sind;
sowie deren pharmazeutisch verträgliche Salze.

2. Verbindung der Formel I nach Anspruch 1, **dadurch gekennzeichnet, daß** darin bedeuten:
R(1) H, F, Cl, Alkyl mit 1, 2, 3 oder 4 C-Atomen, Cycloalkyl mit 5 oder 6 C-Atomen oder -Xₐ-CF₃;
X Sauerstoff;
a Null oder 1;
oder
R(1) -SR(10) oder -OR(10);
R(10) Cycloalkyl mit 4, 5 oder 6 C-Atomen, Chinolyl, Isochinolyl, Pyridyl oder Phenyl,
wobei Chinolyl, Isochinolyl und Pyridyl über ein C- oder ein N-Atom ihres Rings gebunden sind,
und wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1) Chinolyl, Isochinolyl oder Pyridyl,
die über ein C- oder N-Atom ihres Rings verknüpft sind,
oder
R(1) -C≡CR(18);
R(18) Phenyl oder Cycloalkyl mit fünf oder 6 C-Atomen;
R(2) und R(3) -O-CO-R(27);
R(27) Alkyl mit 1, 2, 3 oder 4 C-Atomen, Cycloalkyl mit 3, 4, 5 oder 6 C-Atomen, Phenyl, Biphenylyl, Naphthyl, Pyridyl oder Chinolinyl,
wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, CF₃, Methyl und Methoxy;
wobei stets einer der Substituenten R(2) und R(3)
definiert ist wie R(1);
R(4) und R(5) unabhängig voneinander Methyl, Methoxy, F, Cl, CN oder CF₃.

3. Verbindung der Formel I nach Ansprüchen 1 bis 2, **dadurch gekennzeichnet, daß** darin bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder CF₃;
einer der Substituenten R(2) und R(3)
-O-CO-CH₃; und
der jeweils andere der Substituenten R(2) und R(3)
definiert ist wie R(1);
R(4)und R(5) Methyl, Methoxy, F, Cl, CN oder CF₃.

4. Verfahren zur Herstellung der Verbindung I nach Anspruch 1, **dadurch gekennzeichnet, daß** man eine Verbindung der Formel II 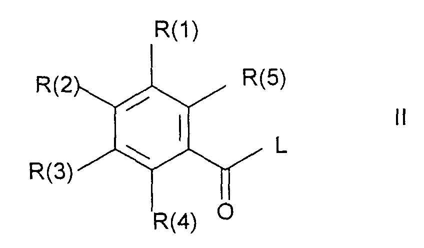 worin R(1) bis R(5) die in Anspruch 1 angegebene Bedeutung besitzen und L für eine leicht nucleophil substituierbare Fluchtgruppe steht, mit Guanidin umsetzt.

5. Verwendung einer Verbindung I nach Anspruch 1 zur Herstellung eines Medikaments zu Behandlung oder Prophylaxe von durch ischämische Zustände verursachten Krankheiten.

6. Verwendung einer Verbindung I nach Anspruch 1 zur Herstellung eines Medikaments zur Behandlung oder Prophylaxe des Herzinfarkts und von Arrhythmien.

7. Verwendung einer Verbindung I nach Anspruch 1 zur Herstellung eines Medikaments zur Behandlung oder Prophylaxe der Angina Pectoris.

8. Verwendung einer Verbindung I nach Anspruch 1 zur Herstellung eines Medikaments zur Behandlung oder Prophylaxe von ischämischen Zuständen des Herzens.

9. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

10. Verwendung einer Verbindung I nach Anspruch 1 zur Herstellung eines Medikaments zur Behandlung oder Prophylaxe von ischämischen Zuständen peripherer Organe und Gliedmaßen.

11. Verwendung einer Verbindung I nach Anspruch 1 zur Herstellung eines Medikaments zur Behandlung von Schockzuständen.

12. Verwendung einer Verbindung I nach Anspruch 1 zur Herstellung eines Medikaments zum Einsatz bei chirurgischen Operationen und Organtransplantationen.

13. Verwendung einer Verbindung I nach Anspruch 1 zur Herstellung eines Medikaments zur Konservierung und Lagerung von Transplantaten für chirurgische Maßnahmen.

14. Verwendung einer Verbindung I nach Anspruch I zur Herstellung eines Medikaments zur Behandlung von Krankheiten, bei denen die Zellproliferation eine primäre oder sekundäre Ursache darstellt.

15. Verwendung einer Verbindung I nach Anspruch 1 zur Herstellung eines Medikaments zur Behandlung oder Prophylaxe von Störungen des Fettstoffwechsels.

16. Arzneimittel, enthaltend eine wirksame Menge einer Verbindung I nach einem oder mehreren der Ansprüche 1 bis 4.

## Claims

1. An ortho-substituted benzoylguanidine of the formula I 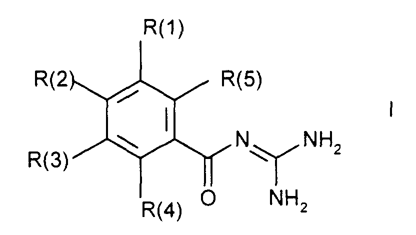 in which:
R(1) is H, F, Cl, Br, I, CN, NO₂, alkyl having 1, 2, 3, 4, 5, 6, 7 or 8 carbon atoms, alkoxy having 1, 2, 3, 4, 5, 6, 7 or 8 carbon atoms, cycloalkyl having 3,4,5,6,7 or 8 carbon atoms, cycloalkoxy having 3, 4, 5, 6, 7 or 8 carbon atoms or Xₐ-(CF₂)_{c}-CF₃;
X is oxygen or sulfur;
a is zero or 1;
c is zero, 1, 2 or 3;
or
R(1) is -SR(10) or -OR(10);
R(10) is -C_{f}H_{2f}-cycloalkyl having 3, 4, 5, 6, 7 or 8 carbon atoms in the cycloalkyl ring, heteroaryl having 1, 2, 3, 4, 5, 6, 7, 8 or 9 carbon atoms or phenyl, where heteroaryl and phenyl are unsubstituted or are substituted by 1-3 substituents selected from the group consisting of F, Cl, CF₃, CH₃, methoxy, hydroxyl, amino, methylamino and dimethylamino;
f is zero or 1;
or
R(1) is phenyl, naphthyl, biphenylyl or heteroaryl having 1, 2, 3, 4, 5, 6, 7, 8 or 9 carbon atoms, the latter linked via a carbon or nitrogen atom of the ring,
each of which is unsubstituted or is substituted by 1-3 substituents selected from the group consisting of F, Cl, CF₃, CH₃, methoxy, hydroxyl, amino, methylamino and dimethylamino,
or
(R1) is -SR(13), -OR(13), -NHR(13), -NR(13)R(14), -C≡CR(18) or -C[R(19)]=CHR(18),
R(13) and R(14), identically or differently, are -(CH₂)_{g}-(CHOH)ₕ-(CH₂)ᵢ-(CHOH)
ₖₖ-R(17) or
-(CH₂)_{g}-O-(CH₂-CH₂O)ₕ-R(24);
R(17) is hydrogen or methyl,
g, h and i, identically or differently, are zero, 1 or 2;
kk is 1 or 2;
R(24) is H, alkyl having 1, 2, 3, 4, 5 or 6 carbon atoms, cycloalkyl having 3, 4, 5, 6, 7 or 8 carbon atoms or-CₘH₂ₘ-R(18);
m is 1, 2, 3 or 4;
R(18) is phenyl,
which is unsubstituted or is substituted by 1-3 substituents selected from the group consisting of F, Cl, CF₃, methyl, methoxy and NR(25)R(26);
R(25) and R(26) are
H or alkyl having 1, 2, 3 or 4 carbon atoms;
or
R(18) is heteroaryl having 1, 2, 3, 4, 5, 6, 7, 8 or 9 carbon atoms, which is unsubstituted or is substituted as phenyl;
or
R(18) is cycloalkyl having 3, 4, 5, 6, 7 or 8 carbon atoms;
R(19) is hydrogen or methyl;
one of the substituents R(2) and R(3) is
-O-CO-R(27);
R(27) is alkyl having 1, 2, 3 or 4 carbon atoms, cycloalkyl having 3, 4, 5 or 6 carbon atoms, phenyl, biphenylyl, naphthyl, pyridyl or quinolinyl,
where phenyl, biphenylyl, naphthyl, pyridyl or quinolinyl are unsubstituted or substituted by 1-3 substituents selected from the group consisting of F, Cl, CF₃, methyl and methoxy;
and
the other of the substituents R(2) and R(3) in each case
is defined as R(1);
R(4) and R(5) are hydrogen, alkyl having 1 or 2 carbon atoms, alkoxy having 1 or 2 carbon atoms, F, Cl, CN or -(CF₂)ₒ-CF₃;
o is zero or 1,
where R(4) and R(5) are not simultaneously hydrogen.
or its pharmaceutically tolerable salts.

2. A compound of the formula I as claimed in claim 1, wherein:
R(1) is H, F, Cl, alkyl having 1, 2, 3 or 4 carbon atoms, cycloalkyl having 5 or 6 carbon atoms or -Xₐ-CF₃;
X is oxygen;
a is zero or 1;
or
R(1) is -SR(10) or -OR(10);
R(10) is cycloalkyl having 4, 5 or 6 carbon atoms, quinolyl, isoquinolyl, pyridyl or phenyl,
quinolyl, isoquinolyl and pyridyl being bonded via a carbon or nitrogen atom of their ring, and phenyl being unsubstituted or substituted by 1-3 substituents selected from the group consisting of F, Cl, CF₃, CH₃, methoxy, hydroxyl, amino, methylamino and dimethylamino;
or
R(1) is quinolyl, isoquinolyl or pyridyl
each of which is linked via a carbon or nitrogen atom of their ring,
or
(R1) is -C≡CR(18);
R(18) is phenyl or cycloalkyl having five or 6 carbon atoms,
R(2) and R(3) are -O-CO-R(27);
R(27) is alkyl having 1, 2, 3 or 4 carbon atoms, cycloalkyl having 3, 4, 5 or 6 carbon atoms, phenyl, biphenylyl, naphthyl, pyridyl or quinolinyl,
where phenyl, biphenylyl, naphthyl, pyridyl or quinolinyl are unsubstituted or substituted by 1-3 substituents selected from the group consisting of F, Cl, CF₃, methyl and methoxy;
where one of the substituents R(2) and R(3)
is always defined as R(1);
R(4) and R(5) independently of one another are methyl, methoxy, F, Cl, CN or CF₃.

3. A compound of the formula I as claimed in claims 1 to 2, wherein:
R(1) is H, F, Cl, alkyl having 1, 2, 3 or 4 carbon atoms, alkoxy having 1, 2, 3 or 4 carbon atoms, cycloalkyl having 5 or 6 carbon atoms, cycloalkoxy having 5 or 6 carbon atoms or CF₃;
one of the substituents R(2) and R(3) is
-O-CO-CH₃;
and
the other of the substituents R(2) and R(3) in each case is defined as R(1):
R(4) and R(5) are methyl, methoxy, F, Cl, CN or CF₃.

4. A process for preparing the compound I as claimed in claim 1, which comprises reacting a compound of the formula II 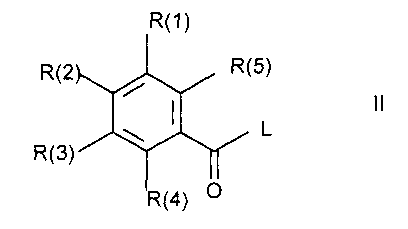 in which R(1) to R(5) have the meaning given in claim 1 and L is a leaving group which can readily be substituted nucleophilically, with guanidine.

5. The use of a compound I as claimed in claim 1 for preparing a medicament for treatment or prophylaxis of illnesses caused by ischemic conditions.

6. The use of a compound I as claimed in claim 1 for preparing a medicament for the treatment or prophylaxis of cardiac infarct and of arrhythmias.

7. The use of a compound I as claimed in claim 1 for preparing a medicament for the treatment or prophylaxis of angina pectoris.

8. The use of a compound I as claimed in claim 1 for preparing a medicament for the treatment or prophylaxis of ischemic conditions of the heart.

9. The use of a compound I as claimed in claim 1 for preparing a medicament for the treatment or prophylaxis of ischemic conditions of the peripheral and central nervous system and of stroke.

10. The use of a compound I as claimed in claim 1 for preparing a medicament for the treatment or prophylaxis of ischemic conditions of peripheral organs and limbs.

11. The use of a compound I as claimed in claim 1 for preparing a medicament for the treatment of states of shock.

12. The use of a compound I as claimed in claim 1 for preparing a medicament for employment in surgical operations and organ transplantations.

13. The use of a compound I as claimed in claim 1 for preparing a medicament for the preservation and storage of transplants for surgical measures.

14. The use of a compound I as claimed in claim 1 for preparing a medicament for the treatment of diseases in which cell proliferation is a primary or secondary cause.

15. The use of a compound I as claimed in claim 1 for preparing a medicament for the treatment or prophylaxis of disorders of lipid metabolism.

16. A medicine, comprising an effective amount of a compound I as claimed in one or more of claims 1 to 4.

## Revendications

1. Benzoylguanidines ortho-substituées de la formule I 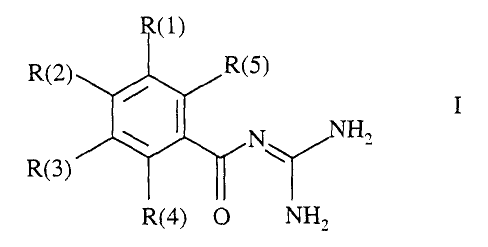 où
R(1) représente H, F, Cl, Br, I, CN, NO₂, un groupe alkyle avec 1, 2, 3, 4, 5, 6, 7 ou 8 atomes-C, alcoxy avec 1, 2, 3, 4, 5, 6, 7 ou 8 atomes-C, cycloalkyle avec 3, 4, 5, 6, 7 ou 8 atomes-C, cycloalcoxy avec 3, 4, 5, 6, 7 ou 8 atomes-C ou Xₐ-(CF₂)_{c}-CF₃;
X représente l'oxygène ou le soufre;
a est égal à 0 ou 1;
c est égal à 0, 1, 2 ou 3;
ou
R(1) représente -SR(10) ou -OR(10);
R(10) représente un groupe -C_{f}H_{2f}-cycloalkyle avec 3, 4, 5, 6, 7 ou 8 atomes-C dans le cycle cycloalkyle, hétéroaryle avec 1, 2, 3, 4, 5, 6, 7, 8 ou 9 atomes-C ou phényle,
les groupes hétéroaryle et phényle étant non substitués ou substitués avec 1-3 substituants choisis parmi F, CI, CF₃, CH₃, le groupe méthoxy, hydroxy, amino, méthylamino et diméthylamino;
f est égal à 0 ou 1;
ou
R(1) représente un groupe phényle, naphtyle, biphénylyle ou hétéroaryle avec 1, 2, 3, 4, 5, 6, 7, 8 ou 9 atomes-C, dont le dernier est lié par l'intermédiaire d'un atome-C ou -N du cycle,
lesquels sont non substitués ou substitués avec 1-3 substituants choisis parmi F, Cl, CF₃, CH₃, le groupe méthoxy, hydroxy, amino, méthylamino et diméthylamino,
ou
R(1) représente -SR(13), -OR(13), -NHR(13), -NR(13)R(14), -C≡CR(18) ou
-C[R(19)]=CHR(18);
R(13) et R(14) représentent identiquement ou différemment -(CH₂)_{g}-(CHOH)ₕ-(CH₂)ᵢ-(CHOH)ₖₖ-R(17) ou -(CH₂)_{g}-O-(CH₂-CH₂O)ₕ-R(24);
R(17) représente l'hydrogène ou le groupe méthyle;
g, h et i sont identiquement ou différemment égaux à 0, 1 ou 2;
kk est égal à 1 ou 2;
R(24) représente H, un groupe alkyle avec 1, 2, 3, 4, 5 ou 6 atomes-C, cycloalkyle avec 3, 4, 5, 6, 7 ou 8 atomes-C ou -CₘH₂ₘ-R(18);
m est égal à 1, 2, 3 ou 4;
R(18) représente le groupe phényle,
lequel est non substitué ou substitué avec 1-3 substituants choisis parmi F, Cl, CF₃, le groupe méthyle, méthoxy et NR(25)R(26); R(25) et R(26) représentent H ou un groupe alkyle avec 1, 2, 3 ou 4 atomes-C; ou
R(18) représente un groupe hétéroaryle avec 1, 2, 3, 4, 5, 6, 7, 8 ou 9 atomes de carbone,
lequel est non substitué ou est substitué comme le groupe phényle; ou
R(18) représente un groupe cycloalkyle avec 3, 4, 5, 6, 7 ou 8 atomes-C;
R(19) représente l'hydrogène ou le groupe méthyle;
un des substituants R(2) et R(3) représente -O-CO-R(27);
R(27) représente un groupe alkyle avec 1, 2, 3 ou 4 atomes-C, cycloalkyle avec 3, 4, 5 ou 6 atomes-C, phényle, biphénylyle, naphtyle, pyridyle ou quinolinyle, les groupes phényle, biphénylyle, naphtyle, pyridyle ou quinolinyle étant non substitués ou substitués avec 1-3 substituants choisis parmi F, Cl, CF₃, le groupe méthyle et méthoxy;
et
l'autre des substituants R(2) et R(3) est à chaque fois défini comme R(1);
R(4) et R(5) représentent l'hydrogène, un groupe alkyle avec 1 ou 2 atomes-C, un groupe alcoxy avec 1 ou 2 atomes-C, F, Cl, CN ou -(CF₂)ₒ-CF₃;
o est égal à 0 ou 1
R(4) et R(5) n'étant pas simultanément l'hydrogène;
ainsi que leurs sels pharmaceutiquement compatibles.

2. Composé de la formule I selon la revendication 1, **caractérisé en ce que** :
R(1) représente H, F, Cl, un groupe alkyle avec 1, 2, 3 ou 4 atomes-C, cycloalkyle avec 5 ou 6 atomes-C ou -Xₐ-CF₃;
X représente l'oxygène;
a est égal à 0 ou 1;
ou
R(1) représente -SR(10) ou -OR(10);
R(10) représente un groupe cycloalkyle avec 4, 5 ou 6 atomes-C, quinolyle, isoquinolyle, pyridyle ou phényle,
les groupes quinolyle, isoquinolyle et pyridyle étant liés par l'intermédiaire d'un atome-C ou -N de leur cycle,
et le groupe phényle étant non substitué ou substitué avec 1-3 substituants choisis parmi F, Cl, CF₃, CH₃, le groupe méthoxy, hydroxy, amino, méthylamino et diméthylamino;
ou
R(1) représente le groupe quinolyle, isoquinolyle ou pyridyle,
lesquels sont liés par l'intermédiaire d'un atome-C ou -N de leur cycle,
ou
R(1) représente -C≡CR(18);
R(18) représente le groupe phényle ou cycloalkyle avec 5 ou 6 atomes-C;
R(2) et R(3) représentent -O-CO-R(27);
R(27) représente un groupe alkyle avec 1, 2, 3 ou 4 atomes-C, cycloalkyle avec 3, 4, 5 ou 6 atomes-C, phényle, biphénylyle, naphtyle, pyridyle ou quinolinyle, les groupes phényle, biphénylyle, naphtyle, pyridyle ou quinolinyle étant non substitués ou substitués avec 1-3 substituants choisis parmi F, Cl, CF₃, le groupe méthyle et méthoxy;
un des substituants R(2) et R(3) étant toujours défini comme R(1);
R(4) et R(5) représentent indépendamment le groupe méthyle, méthoxy, F, Cl, CN ou CF₃.

3. Composé de la formule I selon les revendications 1 à 2, **caractérisé en ce que** :
R(1) représente H, F, Cl, un groupe alkyle avec 1, 2, 3 ou 4 atomes-C, alcoxy avec 1, 2, 3 ou 4 atomes-C, cycloalkyle avec 5 ou 6 atomes-C, cycloalcoxy avec 5 ou 6 atomes-C ou CF₃;
un des substituants R(2) et R(3) représente -O-CO-CH₃;
et
l'autre des substituants R(2) et R(3) est à chaque fois défini comme R(1);
R(4) et R(5) représentent le groupe méthyle, méthoxy, F, Cl, CN ou CF₃.

4. Procédé pour la préparation du composé I selon la revendication 1, **caractérisé en ce que** l'on fait réagir avec de la guanidine un composé de la formule II 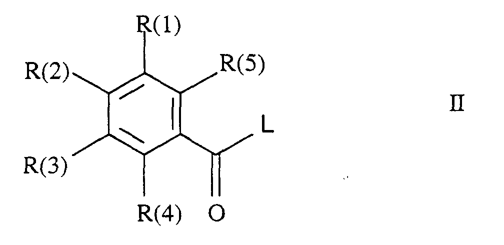 où R(1) à R(5) ont les significations indiquées dans la revendication 1 et L représente un groupe volatil pouvant être facilement nucléophilement substitué.

5. Utilisation d'un composé I selon la revendication 1 pour la préparation d'un médicament pour le traitement ou la prophylaxie de maladies occasionnées par des états ischémiques.

6. Utilisation d'un composé I selon la revendication 1 pour la préparation d'un médicament pour le traitement ou la prophylaxie de l'infarctus du myocarde et d'arythmies.

7. Utilisation d'un composé I selon la revendication 1 pour la préparation d'un médicament pour le traitement ou la prophylaxie de l'angine de poitrine.

8. Utilisation d'un composé I selon la revendication 1 pour la préparation d'un médicament pour le traitement ou la prophylaxie d'états ischémiques du coeur.

9. Utilisation d'un composé I selon la revendication 1 pour la préparation d'un médicament pour le traitement ou la prophylaxie d'états ischémiques du système nerveux périphérique et central et de l'attaque.

10. Utilisation d'un composé I selon la revendication 1 pour la préparation d'un médicament pour le traitement ou la prophylaxie d'états ischémiques d'organes périphériques et des membres.

11. Utilisation d'un composé I selon la revendication 1 pour la préparation d'un médicament pour le traitement d'états de choc.

12. Utilisation d'un composé I selon la revendication 1 pour la préparation d'un médicament destiné à être utilisé dans des opérations chirurgicales et des transplantations d'organes.

13. Utilisation d'un composé I selon la revendication 1 pour la préparation d'un médicament pour la conservation et le stockage de transplantations destinées à des mesures chirurgicales.

14. Utilisation d'un composé I selon la revendication 1 pour la préparation d'un médicament pour le traitement de maladies, dans lesquelles la prolifération des cellules est une cause primaire ou secondaire.

15. Utilisation d'un composé I selon la revendication 1 pour la préparation d'un médicament pour le traitement ou la prophylaxie des troubles du métabolisme des graisses.

16. Médicament contenant une quantité efficace d'un composé I selon l'une ou plusieurs des revendications 1 à 4.
